# EUROPEAN PATENT SPECIFICATION

(11) **EP 1 694 399 B1**
(45) Date of publication and mention of the grant of the patent: **02.11.2011**
(21) Application number: 04809058.3
(22) Date of filing: 15.12.2004
(51) Int. Cl.: A61M 25/10, A61M 16/04, A61L 2/16

(54) **DEVICE FOR ADMINISTERING THERAPEUTIC AGENTS**
VORRICHTUNG ZUR VERABREICHUNG VON THERAPEUTISCHEN MITTELN
DISPOSITIF D'ADMINISTRATION D'AGENTS THÉRAPEUTIQUES

(30) Priority: 15.12.2003 SE 0303364; 11.06.2004 SE 0401498
(43) Date of publication of application: 30.08.2006
(73) Proprietor: Nitricare HB, 757 57 Uppsala (SE)
(72) Inventor: LUNDBERG, Jon, 182 62 DJURSHOLM (SE); WEITZBERG, Eddie, 114 46 STOCKHOLM (SE)
(74) Representative: Holmberg, Martin Tor
(86) International application number: PCT/SE2004/001879
(87) International publication number: WO 2005/056102

(56) References cited:
- WO-A1-84/01721
- US-A- 5 417 657
- US-A1- 2002 082 221
- CARLSSON S. ET AL: 'Effects of pH, Nitrite, and Ascorbic Acid on Nonenzymatic Nitric Oxide Generation and Bacterial Growth in Urine' NITRIC OXIDE: BIOLOGY AND CHEMISTRY vol. 5, no. 6, 2001, pages 580 - 586, XP002985289

## Description

The present invention concerns a device for administering therapeutic agents, e.g. to be used for for the treatment or prevention of a disease, such as but not limited to infectious diseases, inflammatory diseases, cancer etc., or for preventing or reducing the Incidence of nosocomial infections in humans and animals having an invasive medical device inserted into the body, in particular catheter-associated urinary tract infections. More specifically, the invention relates to a device having means for releasing low molecular therapeutic agents, such as anticancer drugs, anti-inflammatory, antiviral or antimicrobial compounds that permeate to the adjacent tissue and/or body cavity.

### Background

In the field of medicine, a distinction can be made between local and systemic treatments, and between invasive and non-invasive Interventions, in the prevention, treatment or alleviation of an abnormal function or state involving any structure, part or system of a living organism. Systemic administration of a drug does inevitably exert certain effects or side effects also on other organs or functions than those subject to the treatment. Likewise, a surgical intervention always causes an extent of trauma to surrounding, healthy tissue, and in the case of larger surgical operations, constitutes a considerable strain on the entire organism.

Nosocomial infections are infections that are caught during a treatment or procedure performed in a hospital. Nosocomial infections are a major global concern that leads to increased hospitalisation, and sometimes even permanent debilities for the patient. In addition to the consequences for the patient, nosocomial infections may infect other patients and cause increased health care and hospital costs.

Major sources of nosocomial infections are insufficient sterilisation of medical equipment and unsatisfactory hygiene of the personnel at hospitals and other care centres and nursing homes. Outbreaks of nosocomial infection have been linked to a variety of non-sterile equipment like electronic thermometers and blood pressure cuffs.

*Staphylococcus aureus* is the most common cause of nosocomial infections and is of increasing concern because of the spread of methicillin resistant strains, which are refractory to treatment by most antibiotics. Non-limiting examples of other microbial pathogens causing nosocomial infections are *Escherichia coli, Serratia marcescens, Klebsiella* and *Enterobacter* species, and *Candida* species.

Venous catheters, urinary catheters, and ventilator-associated tubing are common sites and causes of nosocomial infections. Actually, catheter-associated urinary tract infection (CAUTI) is the most common nosocomial infection and is, together with nosocomial pneumonia, two of the major causes of hospital-acquired infection, for which a substantial proportion of prescribed antibiotics is used.

Urinary catheterisation is a routine procedure in the hospital and chronic care settings and is associated with a significant risk of infection. It is estimated that bacteriuria develops with a daily incidence of around 5 - 10 %. The presence of a catheter within the urinary tract may also increase the difficulty of treating the infection. If a urinary catheter is left in place for long periods of time, bacteria will inevitably grow in it. A harmful infection may occur if the number of bacteria becomes large or if specific pathologic bacteria grow in the urinary tract. Complications of CAUTI include bacteraemia, pyelonephritis, urinary stones and renal failure with resulting morbidity and increased risk of death.

Most microorganisms, except *Staphylococcus aureus,* causing endemic CAUTI derive from the patient's own colonic and perineal flora or from the hands of healthcare personnel during catheter insertion or manipulation of the collection system. Extraluminal contamination may occur early when the catheter is inserted or later by microorganisms ascending from the perineum by capillary action in the thin mucous film close to the external catheter surface. Intraluminal contamination occurs by reflux of microorganisms gaining access to the catheter lumen due to breakage in the closed drainage or contamination of collection bag urine.

Most urinary catheters are equipped with an inflatable cuff situated near the catheter tip. The cuff keeps the catheter in place in the bladder after insertion. When inflated it represents the largest surfaces area of the catheter in the bladder. The outflow orifice of the catheter is situated distal to the cuff meaning that the cuff itself will be constantly embedded in residual urine. It is well known that urine is an excellent growth medium for urinary pathogens. Consequently, the cuff is a particularly vulnerable site for bacterial adherence and growth.

There is a heavy use of systemic antimicrobial drugs to treat and prevent nosocomial infections. Antimicrobial drugs probably keeps the rate of hospital-acquired infections at a considerably lower rate than it would be otherwise, but it unfortunately selects for resistant microorganisms causing many of the most severe nosocomial infections.

Thus, there is a great need for new methods to prevent the occurrence and spread of nosocomial infections, both in an econornical perspective but also from the patient's point of view.

Regarding the treatment, prevention or alleviation of other diseases, such as but not limited to bacterial and viral infections, inflammatory states, cancer etc, among these notably cancer, there is constantly a need for alternative methods and devices, exhibiting better efficacy and reduced side-effects, including trauma and stress resulting form the invasive character of the treatment.

### Prior art

Systemic administration of drugs often causes side effects on organs and tissue not intended for treatment. Local administration of therapeutic agents leading to a more controlled release and treatment is therefore desirable.

US Patent No. 5,007,897 relates to a system for long-term delivery of a liquid medication to hollow body organs, particularly for the treatment of the prostate gland. Said patent describes a urinary catheter comprising, distal to the retention balloon, a permeable membrane bag for drug delivery. The permeable membrane bag is composed of materials capable of slow release drugs from a solution, such as cellulosic and acrylic materials, polyurethane and polyvinyl pyrrolidone and polycarbonate.

Most urinary catheters, when they get infected while they are in use, are covered by a thick biofilm containing the infecting microorganisms embedded in a matrix of host proteins and microbial exoglycocalyx. Bio films may appear both intraluminally and extraluminally. Anti-infective-impregnated and silver-hydrogel catheters, which inhibit adherence of microorganisms to the catheter surface, significantly reduce the risk of CAUTI. However, those coated catheters mainly affect the infections caused by Gram positive organisms or yeasts adhering to the surface of the catheter. Silver alloy and silver oxide coated catheters are also widely used to inhibit establishment of infection.

Other attempts to inhibit CAUTI are the use of anti-infective lubricants when inserting the catheter, soaking the catheter in anti-infective solution before insertion and continuously irrigating the catheterised bladder with anti-infective solution. Efforts have also been made to seal the connection between the catheter and the collection tubing. None of these methods have given satisfactory results and new better ways to inhibit nosocomial infections would be valuable to replace or complement existing methods.

Nitric oxide (NO) is known for its role in the defence against microorganisms.For example, US Patent Application No. 2002/0155174 describes the use of acidified nitrite as an antimicrobial agent for the treatment of viral diseases of the skin by topical application thereto. Acidified nitrite forms nitrous acid, which in turn dissociates to form oxides of nitrogen.

Nablo and Schoenfisch (J Biomed Mater Res, 67A: 1276-1283, 2003) also utilises the antibacterial properties of nitric oxide. They describe NO-releasing sol-gel coatings decreasing bacterial adhesion by 30 to 95% that, for example, can be used on implanted medical devices to prevent biofilm formation.

None of the above mentioned methods for preventing CAUTI relate to the cuff and preventing biofilm formation and bacterial growth on the surface of the cuff. For example, in silver coated catheters the inflatable cuff may be difficult to coat and may remain a particularly susceptible site for infection.

US patent No. 5,417,657 describes a urinary drainage catheter comprising a microporous bacteriostasis balloon for the release of a pharmaceutical agent in order to kill and prevent bacterial growth within and around the bladder. The pharmaceutical agent is released from the bacteriostasis balloon into and around the bladder by osmotic diffusion. The pharmaceutical agents intended for said urinary catheter are, for example, antibiotics such as neosporin, recephin, bactericidin or an antibody. The fact that the release of the antibiotics is driven by osmosis renders the antibacterial effect difficult to control. The water content of the urine is highly variable depending on the present fluid balance of the body, which can lead to an uncontrolled and uneven distribution of the antibiotics. Furthermore, the release of the antibiotic to the outside of the bladd er will be a slow process, exposing the bacteria to the antibiotic during a prolonged time. This increases the risk of development of antibiotic resistant bacteria. With the increased number of bacteria becoming resistant to antibiotics in the industrialized world, long term release of antibiotics should be avoided.

One aim underlying the present invention is to make available methods and devices for local treatment of various conditions, however involving reduced trauma and strain compared to conventional treatments.

Another aim is to make available methods and devices for prevention of hospital acquired infections, such as CAUTI.

In the present invention a device having means for releasing low molecular drugs are used to achieve an effective means to treat or prevent establishment of abnormal states in or around hollow organs of the human or animal body.

### Summary of the invention

The present Invention relates to a device for insertion in a human or animal body and/or body cavity according to claim 1, said device having inflatable/expandable means. The inflatable/expandable means comprises at least one component capable of releasing at least one low molecular drug, said low molecular drug permeating to the adjacent tissue and/or body cavity. In particular, the invention relates to a device having inflatable/expandable means comprising at least one component capable of releasing at least one low molecular antimicrobial compound (LMAC).

Methods for preventing, treating or alleviating various diseases, such as infectious and inflammatory diseases, cancer etc. are also described. In particular, a method for the prevention and treatment of nosocomial infections, such as CAUTI, originating from the insertion and/or use of a medical device in a human or animal is disclosed. The method is **characterized in that** at least one component capable of releasing at least one LMAC is present in an inflatable/expandable means of the device or administered into the device. The LMAC is capable of permeating to the adjacent tissue and/or body cavity.

Carlsson et al. (Nitric oxide: Biology and Chemistry, 2001, vol. 5, no. 6, pages 580-586) discuss the bacterial generation of nitrite in urine during urinary tract infections, and show that acidification of nitrite containing urine resulted in the generation of NO.

Further features and embodiments of the invention, as well as the advantages associated therewith, will be apparent to a skilled person upon study of the present description, non-limiting examples and attached claims.

### Short description of the drawings

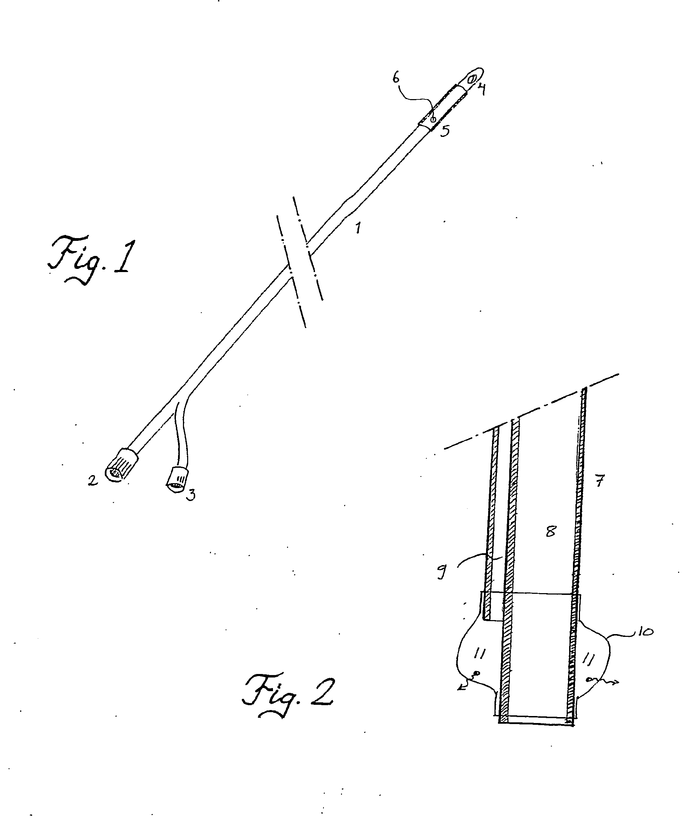

The present invention will be described in closer detail in the following description, examples, and attached drawings, in which
Fig. 1 schematically shows a double lumen urinary catheter having a hollow elongated body 1, an inlet/outlet 2 with a suitable fitting, and an inlet/outlet 3, also with a suitable fitting. The tip of the catheter has an opening 4, which is in fluid connection through a first lumen with the inlet/outlet 2. Slightly below the tip, a cuff 5 is provided. The cuff can be inflated through an opening 6, in fluid connection through a second lumen with the inlet/outlet 3.
Fig. 2 schematically shows the end part of any device 7 to be introduced into the body or a body cavity, and having an optional first lumen 8 for administering fluids, or drugs, or for evacuating body fluids and a second lumen 9 for inflating / deflating a cuff delimited by an elastic membrane 10. The volume of the inflated cuff is indicated as 11. In the alternative, the lumen 8 is solid but flexible, and functions as a guiding means for the device, the main functionality of the device residing in the cuff 11.

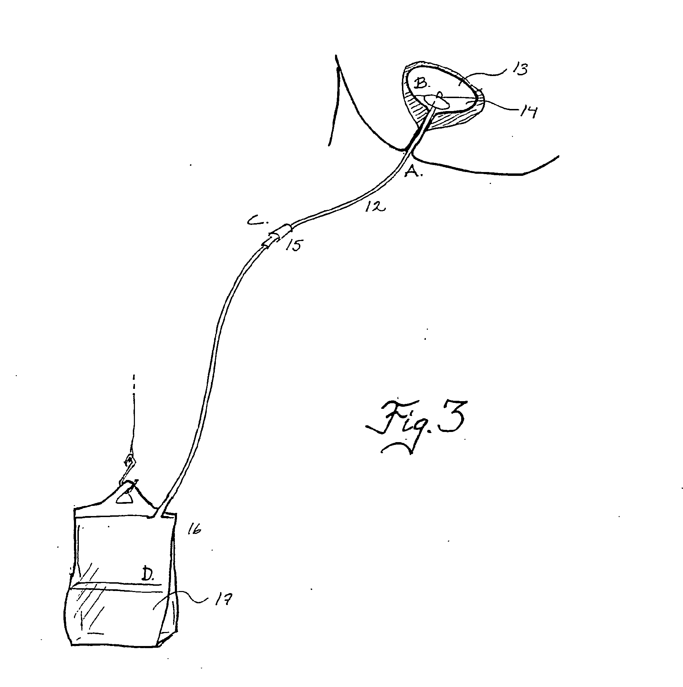

Fig. 3 schematically shows a system for urine collection where a catheter 12 is inserted in a bladder 13. The residual urine volume is indicated as 14. The catheter is connected via connecting means 15 to a collection bag 17, containing a volume of urine indicated as 17. The capital letters A, B, C, and D indicate critical points of contamination and/or bacterial growth.

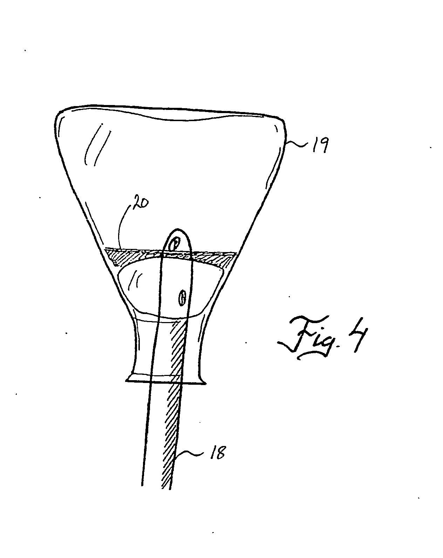

Fig. 4 shows the experimental set-up described in the example, where a sample of *E.*
*coli* infected urine was placed in a 50 ml flask 19. An all-silicone catheter 18 was inserted in the flask, the cuff inflated with a solution according to one embodiment of the invention, and the flask turned up-side-down. The cuff efficiently sealed the neck of the flask and a volume of urine 20 was trapped in the flask. Bacterial growth was monitored by optical density measurements.

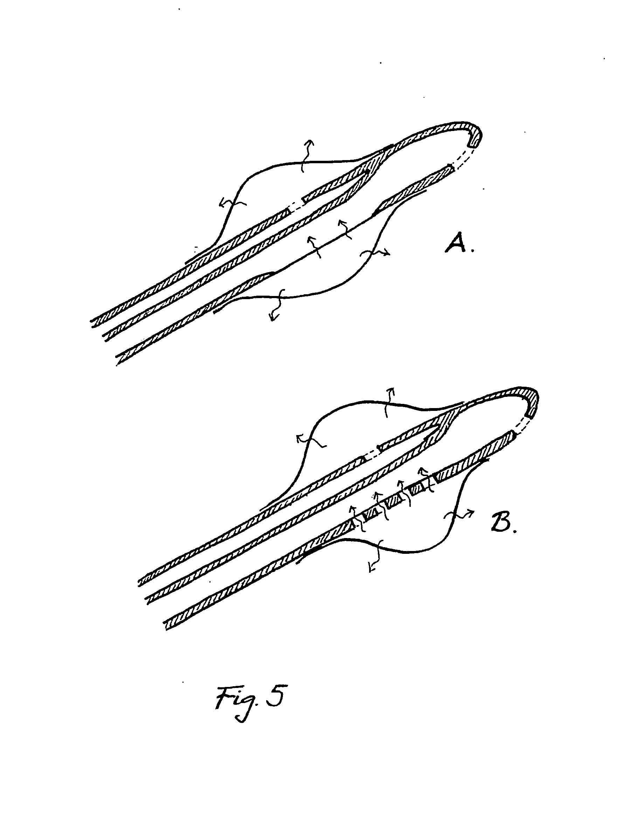

Fig. 5 shows schematically two embodiments where the catheter tip is modified so that the LMAC will penetrate also into the inner lumen of the catheter and thus exerts its antimicrobial effect on the urine collected from the bladder. In A the lumen wall has local areas of reduced thickness, here shown as wells in a cross section of the catheter wall. In B the catheter wall is thinner at the section covered by the cuff, or part of said section.

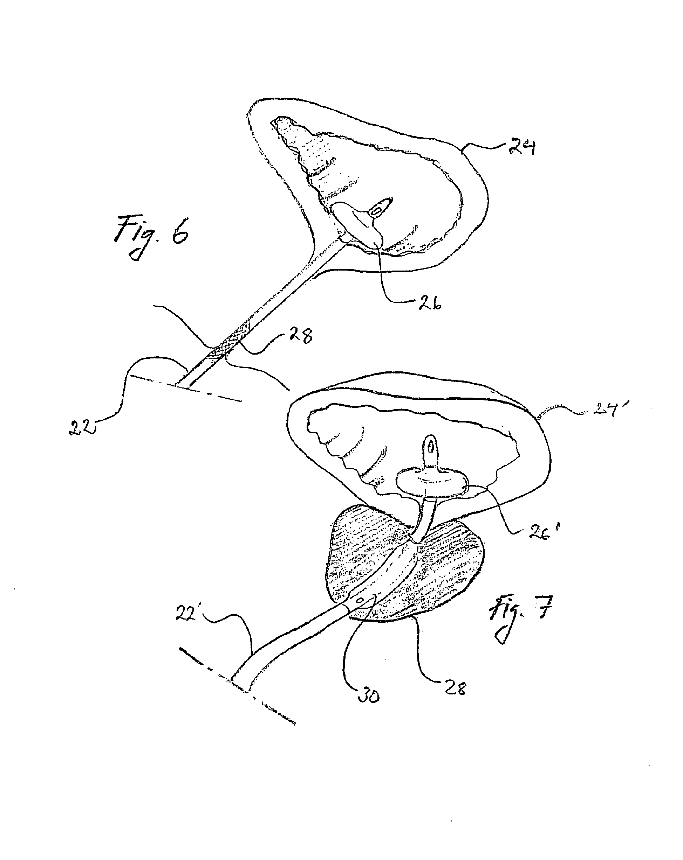

Fig. 6 shows schematically a device according to the invention in place in the urinary tract, depicted according to the female anatomy. The feature illustrated, i.e. the coating 28 of the shaft of the catheter, at the area of the catheter in proximity to the external urethral orifice, is equally applicable to the male anatomy, *mutatis mutandis.*
Fig. 7 shows schematically a device according to the invention in place in the urinary tract, depicted according to the male anatomy. A first inflatable cuff 26 is provided near the tip of the device, aiding in positioning the device so, that a second inflatable section 30 becomes located inside the prostatic urethra.

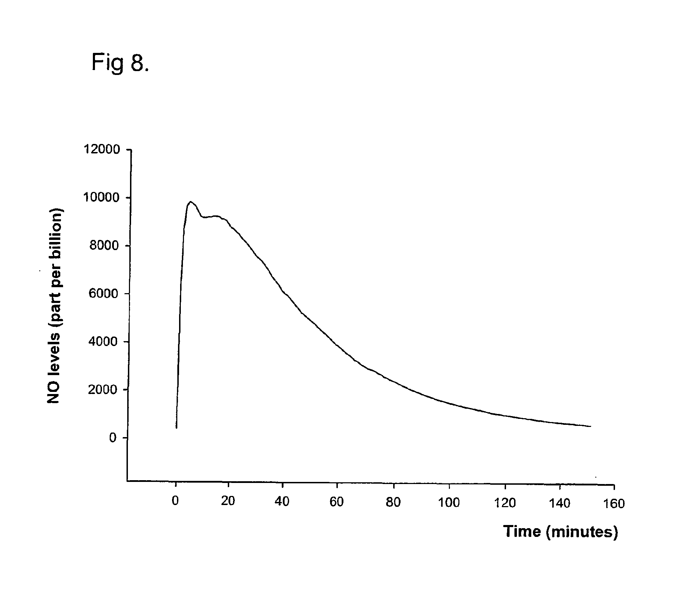

Fig. 8 shows the kinetics of the NO-release from the retention balloon.

### Detailed description of the invention

As mentioned above, local, non-invasive prevention or treatment of an abnormal state in a human or animal body is desirable since systemic administration inevitably leads to higher risks of side effects and surgical operations causes an extent of trauma to surrounding healthy tissue.

Whenever a medical device comes in contact with a patient, a risk of infection always arises. In the case of invasive medical devices, the risk of infection dramatically increases. According to one aspect of the invention, a device and method for reducing the risk of nosocomial infections and for the treatment of nosocomial infections is disclosed. Nosocomial infections may arise both inside and on the outside of an invasive medical device. Thus, the simultaneous reduction and elimination of microbial organisms both on the inside and outside of the device would be beneficial.

The present inventors surprisingly found that the local administration of low molecular drugs can be used for treatment or alleviation of abnormal states in the human or animal body. The present invention therefore relates to a device for local administration of low molecular drugs to hollow organs. In particular low molecular drugs, for example, but not limited to, low molecular antimicrbial compounds (LMAC), can be used in the device for treatment or alleviation of, but not limited to, bacterial infections, viral infection, inflammatory states and cancer. More specifically, the present invention relates to a device for insertion in a human or animal body and/or body cavity, said device having inflatable/expandable means, wherein the inflatable/expandable means comprise or are suitable for receiving at least one component capable of releasing at least one LMAC in the device, said LMAC permeating to the adjacent tissue and/or body cavity.

According to the present invention a low molecular drug is a compound or molecule with sufficiently low molecular weight to permeate the inflatable/expandable means of the inventive device. The low molecular drug has a molecular weight equal to or less than 250 U, preferably equal to or less than 200 U, preferably equal to or less than 150 U, more preferably equal to or less than 100 U, and most preferably equal to or less than 50 U. In one embodiment the low molecular drug is a low molecular antimicrobial compound (LMAC).

### Devices according to the invention

The device of the present invention is an invasive medical device. An invasive medical device refers to any device wherein at least a portion of the device may be inserted percutaneously, through a natural orifice or otherwise into any site of the body of a human or an animal. Examples of medical devices includes urinary catheters, intratracheal tubes, vascular catheters, vascular catheter ports, wound drain tubes, gastric tubes etc. In particular the present invention relates to devices for insertion into the urinary tract, catheters in general, intratracheal tubes for insertion into the respiratory tract of a human or animal body or gastric tubes for insertion into the gastrointestinal tract of a human or animal body.

The device according to the invention comprises inflatable/expandable means. The inflatable/expandable means of the device can be any means for securing a medical device inserted into a human or animal body or body cavity, or means ensuring a tight fit between the device and the surrounding tissue etc. In the present invention the inflatable/expandable means consist of an elastic material of sufficient medical grade being permeable to low molecular compounds, such as a gas, but impermeable to water and which is expandable and inflatable. The inflatable/expandable means can be used to secure the device in the body as in the cuff near the tip of a urinary catheter, or to make sure that there is a tight fit between the device and the surrounding tissue, that the inflatable/expandable means completely fill the space available etc. In particular the inflatable/expandable means is a cuff consisting of, but not limited to, elastic materials comprising polysiloxanes such as silicone rubber comprising polydimethylsiloxanes, latex-free rubber, silicon-coated rubber or a semi-permeable or selectively permeable membrane such as Goretex^{®}. Importantly said membrane allows an low molecular drug, such as, but not limited to, a LMAC, to permeate.

The low molecular drug or the LMAC released in the inflatable/expandable means of the device is a compound capable of permeating said means to the adjacent tissue and/or body cavity thereby exerting its antimicrobial effect both on the inside and on the outside of the device. Examples of low molecular drugs are, but not limited to, low molecular analgetics, anti-inflammatory agents and cytotoxic agents such as, but not limited to, 5-fluoro uracil and cis-platina. Thus, the LMAC can be any antimicrobial compound, such as an antimicrobial gas, with sufficiently low molecular weight to permeate the device. In particular the LMAC is a reactive nitrogen intermediate (RNI), a reactive oxygen intermediate (ROI) or a combination of these two. Examples of LMACs are, but not limited to, nitric oxide (NO), NO₂⁻, N₂O₃, N₂O₄, HNO₂, HNO₃, NO⁺, NO⁻, O₂⁻, O₃, singlet oxygen, H₂O₂, OONO-, HOONO, NOCI, NOSCN, NO thiocyanate, an OH radical and HOCI. Most LMACs, such as, but not limited to, NO, OONO⁻, and NO₂ can also exert cytotoxic effects.

The LMACs exert their antimicrobial effect non-specifically, with multiple cellular targets, which probably reduce the potential of the microorganisms to develop resistance to the LMACs. The use of LMACs with short half-lives in relatively high dose will further reduce the development of resistant bacteria. Furthermore, since some RNIs and ROIs are generated endogenously, the use of such compounds is unlikely to constitute any major health risk to the patient. Another advantage with the present invention is the local production of antibacterial compounds, which will not affect the normal microbial flora of, for example, the gut. The use of antibiotics in the inflatable/expandable means cannot exert the same antimicrobial effect as LMACs since antibiotics cannot permeate said inflatable/expandable means. Furthermore, the LMACs permeating the said inflatable/expandable means will lead to high local concentrations of antimicrobial compounds leading to efficient biofilm prevention.

The LMAC of the present invention consists of or is released from at least one component comprised in the inflatable/expandable means. Said at least one component releasing the LMAC is any compound capable of releasing an antimicrobial compound with sufficiently low molecular weight to permeate the device. Examples of components that release RNIs/ROIs include, but are not limited to, S-nitrosothiols (low or high molecular-weight thiols), NONOates, nitroprusside, organic nitrate/nitrites, inorganic nitrite (NO₂⁻), N-nitroso compounds, C-nitroso compounds, oximes, sydnonimines, oxadiazoles (furoxans), oxatriazoles, nitroxyl generating compounds (e.g. Piloty's acid), hydroxylamine, N-hydroxy-guanidines, nitrosylchloride, sodium azide, nitrosylhydrogensulphate, nitrosyltetrafluoroborat, dinitrosyl-iron-cysteine complex, etc.

There are several factors affecting the release of the LMAC. The RNIs/ROIs released by those compounds have different half-lives and differ in their pH dependency. Thus, the at least one component releasing the LMAC can be alternated depending on the special circumstances associated with the infection, the patient, where in the body the device is inserted etc.

In one embodiment of the invention the low molecular drug, in particular the LMAC, is in a gaseous state at the body temperature of the human or animal into which the inventive device is inserted. The term gaseous state refers to a state of matter distinguished from the solid and the liquid states in which the matter concerned occupies the whole of its container irrespective of its quantity.

The low molecular drug, such as the LMAC, released in the inflatable/expandable means of the inventive method can be both charged and uncharged molecules. Uncharged molecules may show increased permeability of the membrane of the inflatable/expandable means.

Different LMACs exert diverse effects on different bacteria. Thus, the present invention gives the possibility of using different LMACs depending on the infectious bacteria. For example, *S. aureus* is sensitive to NO alone while *E.coli* is not. Furthermore, the LMACs released can also be changed or modified with respect to the localization of the device since, for example, bacteria causing nosocomial infections coupled to the use of a urinary catheter might, at least partly, be distinct from the bacteria causing infections related to the use of an intratracheal tube. Depending on the patient and the type of infection, the dosage of released LMAC can be changed or modified through the present invention. The term "changed or modified" as used above comprises changing the LMAC itself, e.g. by substituting one LMAC for another or using different combinations of LMACs, or by changing the concentration(s) of the same.

The LMAC is released when said at least one component is contacted with a second component. The second component is, an acid initiating the release of the LMAC when contacted with the component releasing the LMAC. There is a big advantage with the release of the LMACs through the contacting of at least two components. Since the LMACs have relatively short half-lives the antibacterial effect will not be reduced during storage or delivery of the compounds.

In another embodiment the component releasing the LMAC and the second component are present in the inflatable/expandable means as dry powders, granulates, thin films or the like coating the inside of the cuff and the contact between the two components is accomplished through the addition of a liquid to said means. The liquid can for example be, but is not limited to, of water, saline or any physiological buffer, such as phosphate buffered saline (PBS) or the like.

In one embodiment of the present invention the at least one component of the device releasing the LMAC releases the LMAC upon acidification. The acidification is achieved through the addition of an acidifying agent (the "second component") reducing the pH in the device to about pH 1 - 5.5, preferably to about 2 - 4. The acidifying agent can be any acid reducing the pH in the device to the above-mentioned pH. Appropriate acids are for example, but not limited to, ascorbic acid, citric acid, acetic acid, butyric acid, diluted hydrochloric acid, diluted sulphuric acid, formic acid, nitrous acid or nitric acid.

In one embodiment of the present invention said at least one component is nitrite and said second component is an acidifying agent that, when brought into contact with nitrite, release RNIs such as nitric oxide, N₂O₃ and nitrous acid. Ascorbic acid is a preferred acidifying agent.

Also described is that the at least one component of the device releases the LMAC releases the LMAC upon alkalinisation. The alkalinisation is achieved through the addition of an agent (the "second component") increasing the pH in the device to a basic pH of about pH 7.5-10. Agents increasing the pH can be, but are not limited to, NaOH, NaCO₃, and NH₃. LMACs that are released upon alkalinisation are for example, but not limited to, nitrosal thiols (S-NO).

In one embodiment of the present invention the Inflatable/expandable means is at least one Inflatable cuff situated somewhere along the device and the at least one component releasing the LMAC is present In said inflatable cuff. In another embodiment the second component is present in said Inflatable cuff. In yet another embodiment both said at least one component releasing a LMAC and the second component are present in said inflatable cuff. The inflatable cuff has an arrangement for introducing said at least one component or said second component in the cuff. Said arrangement of the inflatable cuff making it possible to administer the component(s) repeatedly during the use of the device, thus more efficiently preventing or treating nosocomial infections during prolonged use of the device in a patient. Said arrangement is also suitable for the introduction of a liquid into the cuff contacting the at least one component releasing a LMAC and the second component.

When the device is a catheter (Fig. 3) for insertion into the urinary tract of the human or animal body, the cuff, when the catheter is inserted, is situated in the urinary bladder surrounded by the residual urine present in the bladder. Thus, the LMACs released by the present invention solve the problem with infections establishing on the outside of the cuff surrounded by the residual urine.

In one embodiment of the present invention the part of the device surrounded by the inflatable/expandable means have local areas of reduced thickness in order to allow the LMACs to permeate also to the inside of the device thus exerting an antimicrobial effect also inside the device (Fig. 5A). According to another embodiment the catheter wall is thinner at a section surrounded by or in association with the inflatable cuff or a part thereof (Fig. 5B). The device according to the invention can exert a doubble effect, i. e. both outside the device and inside the device by preventing bacterial infection to spread and migrate to other parts of the device.

According to yet another embodiment, the shaft of the device is partially or entirely coated with a therapeutic agent, e.g. an anti-microbial agent or one or more substances capable of releasing an LMAC as defined above. Fig. 6 shows an embodiment where the part of the device which, when in use, is located at the external urethral orifice, is coated (28). Coated in this context also includes embodiments where one or more substances are incorporated in the material, e.g. embedded in the silicone. While illustrated in relation to the female anatomy, this embodiment is equally applicable to the male anatomy if the longer distance between the external urethral orifice and the bladder is taken into account.

It is also contemplated that one or more metal ions are present in or introduced to the contents of the inflatable/expandable means, or incorporated in the wall of the device, e.g. in the catheter wall or parts thereof, in a concentration sufficient to increase the antimicrobial effect. Incorporation in the wall of the device means that the compounds are either coated on the surface, introduced into, or partially into the material, or evenly mixed in the material. Suitable metal ions are for example ions of Zn, Cu, Mg, Fe, and Ag, available as soluble salts of corresponding metals. According to one embodiment of the invention, also ascorbic acid is used, preferably in combination with a metal ion, and most preferably in combination with Zn. It is preferred that these components are mixed in the material constituting the device, e.g. in the silicone. One advantage of this particular embodiment is that the components can then exert their action both on the outside of the device, e.g. in the urethra or bladder, and inside the device, in the lumen.

It is also contemplated that the device according to the present invention can incorporate features from existing devices, such as but not limited to, urinary catheters known to persons skilled in the art. One such combination is that of a Ag-coated catheter and the inventive LMAC releasing inflatable / expandable means. Other combinations will become evident to persons skilled in the art.

Another embodiment of the invention relates to a device for insertion in a human or animal body and/or body cavity, wherein the device has at least one compound integrated in its material or on the surface thereof, said compound being capable of releasing at least one low molecular drug. When said material or surface is contacted with the body fluids said integrated at least one compound releases at least one low molecular compound, such as, but not limited to, a LMAC.

In one embodiment said device having at least one component integrated into Its material or surface can also release at least one low molecular drug from the inflatable/expandable means as described above.

Examples of compounds that can be integrated into the material or on the surface of the device are, but not limited to, ascorbic acid, nitrite, a reactive nitrogen Intermediate, a reactive oxygen intermediate or any combination thereof. In one embodiment the integrated compound comprises nitrite in combination with ascorbic acid and zinc.

In one embodiment said device having at least one compound integrated in its material or on its surface is a urinary catheter, an intratracheal tube or a gastric tube.

In yet another embodiment said device having at least one compound integrated in its material or on its surface also have the inflatable/expandable means consisting of an elastic material of sufficient medical grade being permeable to low molecular compounds as mentioned above.

### Methods for preventing and/or treating Nosocomial infections

Also disclosed is a method for preventing and/or treating abnormal states in the animal or human body or body cavities through the release of at least one low molecular drug from a device inserted into said body or body cavity. In particular, a method for preventing and/or treating nosocomlal infections originating from the insertion and/or use of a device inserted into a human or animal body and/or body cavity, said device having inflatable/expandable means, wherein at least one component capable of releasing at least one low molecular drug, such as, but not limited to a low molecular antimicrobial compound (LMAC) in said means is administered into said means, said low molecular drug permeates to the adjacent tissue and/or body cavity. The method can be used for reducing the risk of and treating nosocomial infections originating from the insertion and use of a urinary catheter.

In another embodiment the method is used for reducing the risk of and treating nosocomial infections originating from the insertion and use of an intratracheal tube inserted into the respiratory tract of a human or animal body.

In yet another embodiment the method is used for reducing the risk of and treating nosocomial infections originating from the insertion and use of a gastric tube in the gastrointestinal tract of a human or animal body

The inflatable/expandable means of the device can be a means for securing a medical device inserted into a human or animal body or body cavity, or means to ensure a tighter fit between the device and the surrounding tissue, or means for making sure that the entire available volume inside an organ is filled etc. In the invective method the inflatable/expandable means consists of an elastic material of sufficient medical grade being permeable to gas but impermeable to water and which is expandable and inflatable, e.g. to secure the device in the body. In particular the inflatable/expandable means is a cuff consisting of, but not limited to, an elastic material consisting of or comprising polysiloxanes such as silicone rubber comprising polydimethylsiloxanes, latex-free rubber, silicon-coated rubber or a semi-permeable or selectively permeable membrane such as Goretex^{®}. When the device is a urinary catheter the cuff, when inserted into the human or animal body, is situated in the urinary bladder surrounded by the residual urine present in the bladder.

Examples of low molecular drugs are, but not limited to, low molecular analgetics, anti-infiammatory agents and cytotoxic agents such as, but not limited to, 5-fluoro uracil and cis-platina. The low molecular compound exerting the antimicrobial effect can be any antimicrobial compound with sufficiently low molecular weight to permeate the inflatable/expandable means of the device wherein it is released. In particular the LMAC is a RNI, a ROI or a combination of these two. Examples of LMACs are, but not limited to, nitric oxide (NO), NO₂, N₂O₃, N₂O₄, HNO₃, HNO₂, NO⁺, NO⁻, O₂⁻, 03, singlet oxygen, H₂O₂, OONO-, HOONO, an OH radical and HOCI. Most LMACs, such as, but not limited to, NO, OONO⁻, and NO₂ can also exert cytotoxic effects.

The low molecular drug, such as the LMAC, released in the inflatable/expandable means can be both charged and uncharged molecules. Uncharged molecules may show increased permeability of the membrane of the inflatable/expandable means.

It is also contemplated that one or more metal ions are present in or introduced to the contents of the inflatable/expandable means, or incorporated in the wall of the device, e.g. in the catheter wall or parts thereof, in a concentration sufficient to increase the antimicrobial effect. Incorporation in the wall of the device means that the compounds are either coated on the surface, introduced into, or partially into the material, or evenly mixed in the material. Suitable metal ions are for example ions of Zn, Cu, Mg, Fe, and Ag, available as soluble salts of corresponding metals.

Also ascorbic acid can be used, preferably in combination with a metal ion, and most preferably in combination with Zn. It is preferred that these components are mixed in the material constituting the device, e.g. in the silicone. One advantage of this particular embodiment is that the components can then exert their action both on the outside of the device, e.g. in the urethra or bladder, and Inside the device, in the lumen.

The LMAC is released from at least one component comprised in the inflatable/expandable means of the device. Said at least one component releasing the LMAC is any compound capable of releasing an antimicrobial compound with sufficiently low molecular weight to permeate the device. Examples of components that release RNIs/ROIs include, but are not limited to, S-nitrosothiols (low or high molecular-weight thiols), NONOates, nitroprusside, organic nitrate/nitrites, inorganic nitrite (NO₂⁻), N-nitroso compounds, C-nitroso compounds, oximes, sydnonimines, oxadiazoles (furoxans), oxatriazoles, nitroxyl generating compounds (e.g. Piloty's acid), hydroxylamine, N-hydroxy-guanidines, nitrosylchloride, sodium azide, nitrosylhydrogensulphate, nitrosyltetrafluoroborat, dinitrosyl-iron-cysteine complex.

In one embodiment of the methods the LMAC is released when said at least one component is contacted with a second component. The second component can, for example, be a solvent, or a reagent, such as an acid or base initiating the release of the LMAC when contacted with the component releasing the LMAC.

In another embodiment of the method the component releasing the LMAC and the second component are present in the device as dry powders, granulates, thin films or the like and the contact between the two components is accomplished through the addition of a liquid to the device. The liquid can for example be, but is not limited to, water, saline or any physiological buffer, such as phosphate buffered saline (PBS) or the like.

In yet another embodiment of the method said at least one component releasing the LMAC releases the low molecular compound upon acidification. The acidification is achieved through the addition of an acidifying agent reducing the pH in the device to about pH 1 - 5.5, preferably to about 2 - 4. The acidifying agent can be any acid reducing the pH in the device to the above-mentioned pH. Appropriate acids are for example ascorbic acid, citric acid, acetic acid, butyric acid, diluted hydrochloric acid, diluted sulphuric acid, formic acid, nitrous acid, or nitric acid.

In another embodiment of the method said at least one component is nitrite and said second component is an acidifying agent that, when brought into contact with the nitrite, release RNIs such as nitric oxide, N₂O₃ and nitrous acid. Ascorbic acid is a preferred acidifying agent.

In one embodiment of the present method, the at least one component of releasing the LMAC releases the LMAC upon alkalinisation. The alkalinisation is achieved through the addition of an agent (the "second component") increasing the pH In the device to a basic pH of about pH 7.5-10. Agents increasing the pH can be, but are not limited to, NaOH, NaCO₃, and NH₃. LMACs that are released upon alkalinisation are for example, but not limited to, nitrosal thiols (S-NO).

The low molecular compound can be in a gaseous state at the body temperature of the human or animal in to which the device is inserted.

In another embodiment the method is for treatment of nosocomial infections in and around an inflatable cuff situated along the device. The at least one component and/or the second component is/are administered into the cuff situated along the device. The Inflatable cuff has an arrangement (3 and 6 in Fig. 1) making it possible to administer said component that releases at least one LMAC and/or said second component to the cuff. Through the arrangement of the inflatable cuff said component releasing a low molecular compound and/or the second component can be added to the device repeatedly during the use of the device, thus more efficiently preventing or treating nosocomial infections during prolonged use of the device in a patient. Said arrangement is also suitable for the administration of a liquid Into the cuff contacting the at least one component releasing a LMAC and the second component. The acidifying agent can also be administered through said arrangement. Furthermore, through the use of said arrangement it is easy to change or modify the dosage of the released LMAC in each Individual patient.

The cuff is initially inflated with about 10 ml water or saline, or with about 10 ml of a solution comprising at least one component releasing a LMAC and a second component, preferably nitrite and ascorbic acid. In the event that the cuff was initially inflated with water or saline only, an amount of about 2 ml is withdrawn from the cuff, using a syringe containing at least one component releasing a LMAC and the second component, preferably nitrite and ascorbic acid in dry form. The components are dissolved in the water or saline, and the solution introduced in the cuff. The procedure can be repeated at suitable intervals, e.g. when replacing the urine collection bag, or at pre-determined intervals, in order to replenish the concentration of the components in the cuff, and to guarantee an effective antimicrobial effect.

In the alternative, the cuff is initially filled using a syringe containing at least one component releasing a LMAC and the second component, preferably nitrite and ascorbic acid in dry form. This embodiment has the advantage that the normal routines relating to the insertion and maintenance of a catheter need not be changed, it is sufficient that such syringe is used.

Also disclosed is a method for the prevention and treatment of nosocomial infections, wherein a device as described above is used.

Urinary collection devices, such as containers or drainage bags, are generally used for collecting urine from a catheterised patient. A collection device usually has tubing attached to the catheter leading the urine to the collection device (Fig. 3). Catheterisation often results in the possibility of urinary tract infection resulting from growth of microorganisms in the collection device and the associated tubing. The microorganisms growing in the collection device might be transported back to the bladder when the collection system is moved or manipulated in association with breakage in closed drainage. There are four main critical points for establishment of infection during catheterisation as Indicated in Fig. 3. One is the area surrounding the external urethral orifice, indicated as A. Another is the inside of the bladder, B, where the cuff and the tip of the catheter reside in residual urine. A third point of infection is the connection or coupling C between the catheter itself and the collection device. In Fig. 3 the coupling C is drawn as situated closer to the catheter than to the collection bag. In reality, C could be at the collection bag, any where between the catheter and said bag, or in fact, several couplings C could be provided. The fourth critical point is the collection bag itself, D, where bacteria can grow in large volumes of urine at room temperature. There is thus also a need for an effective method for the prevention of urinary tract infection originating from contaminants in the collection system.

Thus, further disclosed is a method for prevention of nosocomial infections in patients having a urinary catheter by preventing microbial growth in the collection device coupled to a catheter wherein at least one component releasing a LMAC is added to the collection device. An advantage with the use of this method is that the use of LMAC, exerting its antimicrobial effect non-specifically, most likely will have lower probability of giving rise to antibiotic resistant microbial strains. In particular the LMAC is a RNI, a ROI or a combination of these two. Examples of LMACs are, but not limited to, nitric oxide (NO), NO₂, N₂O₃, N₂O₄, HNO₃, HNO₂, NO⁺, NO⁻, O₂⁻, O₃, singlet oxygen, H₂O₂, OONO-, HOONO, NOCI, NOSCN, NO thiocyanate, an OH radical and HOCI.

The at least one component releasing the LMAC in the collection device is any compound capable of releasing a LMAC in the collection device, such as, but not limited to, S-nitrosothiols (low or high molecular-weight thiols), NONOates, nitroprusside, organic nitrate/nitrites, inorganic nitrite, N-nitroso compounds, C-nitroso compounds, oximes, sydnonimines, oxadiazoles (furoxans), oxatriazoles, nitroxyl generating compounds (e.g. Piloty's acid), hydroxylamine, N-hydroxy-guanidines, nitrosylchloride, sodium azide, nitrosylhydrogensulphate, nitrosyltetrafluoroborat, dinitrosyl-iron-cysteine complex.

In another embodiment of the method said at least one component releasing the LMAC in the collection device releases the low molecular compound upon acidification. The acidification is achieved through the addition of an acidifying agent ("the second component") reducing the pH in the device to about pH 1 - 5.5, preferably to about 2 - 4. The acidifying agent can be any acid reducing the pH in the device to the above-mentioned pH. Appropriate acids are for example ascorbic acid, citric acid, acetic acid, butyric acid, diluted hydrochloric acid, diluted sulphuric acid, formic acid, nitrous acid, or nitric acid.

In another embodiment of the method said at least one component is nitrite and said second component is an acidifying agent that, when brought Into contact with nitrite, release RNIs such as nitric oxide, N₂O₃ and nitrous acid in the collection device. Ascorbic acid is a preferred acidifying agent.

In one embodiment the device according to the invention or the method as described above is used for treatment or alleviation of an abnormal state in a human or animal body. Said abnormal state being chosen among, but not limited to, bacterial infections, viral infections, inflammatory states, and cancer. The treatment can be performed on the urogenital organs including one of the urethra, the prostate, the bladder, the vagina, cervix, uterus or oviducts.

### A kit of parts

A kit to be used for local delivery of at least one low molecular drug and treatment of abnormal states in the human or animal body is also described. Examples of abnormal states are, but not limited to, bacterial infections, viral infection, inflammatory states and cancer. In particular said kit comprises a device having an inflatable cuff and a syringe suitable for inflating said cuff in a human or animal body or body cavity and said syringe comprises the necessary components for the release of a low molecular drug after administration of said components Into said inflatable cuff of said device.

Further disclosed is a kit to be used for preventing or treating nosocomial infections originating from the insertion and/or use of an invasive medical device, such as a urinary catheter, intratracheal tube or gastric tube, having an inflatable cuff in a human or animal body and/or body cavity. Said kit comprises said device and a syringe suitable for inflating said cuff and said syringe comprises the necessary components for the release of a LMAC after administration of said components into said Inflatable cuff of said device. The LMAC can be any compound with sufficiently low molecular weight to permeate the cuff and/or the device. In one embodiment the LMAC is a reactive nitrogen intermediate (RNI), a reactive oxygen intermediate (ROI) or a combination of these two. The kit is easy to use and there is no need for additional equipment or special training of the personnel at hospitals or other health care centres.

The necessary components comprised in the syringe are at least one component releasing the LMAC and, optionally, a second component inducing the release of the LMAC upon contact with the LMAC releasing component. The component(s) can be present as dry powders, granulates, thin films or the like that release said LMAC upon combination with a liquid such as water, saline or any physiological buffer, such as PBS. In one embodiment the powders and the liquid are kept in separate containers until combined in the syringe prior to administration or simultaneously with the inflation of said cuff.

One of the necessary components in the syringe can be nitrite that upon acidification produces RNIs. The acidification can for example be achieved through the addition of, but not limited to, ascorbic acid, citric acid, acetic acid, butyric acid, diluted hydrochloric acid, diluted sulphuric acid, formic acid, nitrous acid, or nitric acid.

The present invention can also be used in combination with other devices, methods and/or kits developed for the prevention and/or treatment of nosocomial infections, such as, bur not limited to, anti-infective lubricants that can be used when inserting the medical device, anti-infective creams or ointments applied to the device, soaking of the medical device in anti-infective antimicrobial-drug solution prior to use, silver oxide coated devices (e.g. catheters). Similarly, the device can comprise features taken from known devices, e.g. catheters, familiar to persons skilled in the art.

### Treatment of cancer etc.

Another aspect disclosed Is the possibility to treat diseases other than infectious diseases, such as cancer, inside the human or animal body, preferably but not limited to the inside of a hollow organ. In one embodiment the device according to the invention is used for treatment or alleviation of an abnormal state in a human or animal body. Another embodiment relates to the treatment of prostate cancer, according to which the inventive device has a second inflatable / expandable means, capable of releasing an anti-cancer drug into the prostate. It is contemplated that the treatment can be modified by controlling the temperature of the drug inside the device, controlling the pressure the expandable means exerts against the walls of the prostatic urethra etc. The treatment of prostate cancer can constitute an alternative to known treatments or treatments yet to be developed, or preferably a supplement to such treatments. It is contemplated that the prostate can be simultaneously subjected to irradiation and the administration of a drug, and optionally an elevated temperature in order to enhance the effect of the treatment, and possible to enable a reduction of the level of irradiation.

According to another embodiment, bladder cancer is treated using a device and method as described above in relation to the treatment of urinary tract infections, with the modification that an anti-cancer drug is used instead of the LMAC, or together with an LMAC. Reactive nitrogen intermediates (RNIs), and reactive oxygen intermediates (ROIs) as presented above, are contemplated for use also in the treatment of cancer, either alone or In combination with an anti-cancer drug. Also here it is contemplated that the method as described above is used as a supplement to known treatments or treatments yet to be developed.

The present invention also relates to a device as described above exerting its antimicrobial effect on non-nosocomial infections. In one embodiment the at least one component releasing at least one LMAC is present in an inflatable cuff situated along a catheter inserted in the urinary tract of a human. The LMAC penetrates the cuff and exert its effect on the prostate. It has been suggested that for example chronic prostatitis is caused by a bacterial infection. By arranging a second cuff at a specific distance from the first cuff, securing the device, the second cuff can be positioned in the urinary tract so that it is surrounded by the prostate gland. This embodiment makes possible a long term, minimally invaslve treatment of the prostate.

According to yet another embodiment, the inventive device and the above described method is applied to the treatment of vaginal or cervical disorders, for the administration of a drug to the vagina and/or cervix, e.g. for the treatment of viral infections, e.g. herpes virus, human papilloma virus, dysplasia or cancer, e.g. cervical cancer or vaginal cancer. Similarly, a device and method according as disclosed above can be used In the treatment of abnormal conditions of the uterus, for example but not limited to uterine cancer, uterine fibroma, and uterine ischemia.

It is also contemplated that the inventive device and the method can be utilised in procedures like the removal or treatment of thrombosis; e.g. in order to dilate a congested blood vessel and simultaneously administer a drug to the site of dilatation or surgical intervention, e.g. in order to prevent reocculsion of the blood vessel.

### Advantages of the invention

The present Invention relates to a device for local administration of a pharmaceutical agent to a human or animal body or body cavity. Thus, the present invention solves the problems associated with systemic administration of drugs. It offers a local treatment wherein parameters as time, place and concentration easily can be controlled.

The invention offers a simple and safe solution to a serious problem. One particular advantage lies in the low systemic toxicity of the LMACs suggested for use in the inventive device . For example nitrite is generally accepted as a food additive, in particular in cured meat products. The highest concentration allowed is 200 ppm, which equals a concentration of about 3 mM. This is in the same range as the concentration reached according to the invention. Notably, the stomach mucosa is continuously exposed to similar amounts of endogenous nitrogen oxides from natural acidification of nitrite in saliva. The suggested antimicrobial compounds are expected to have low or negligible acute toxicity to host cells.

Even in the unlikely event of cuff rupture, the contents of the cuff (about 10 ml) will be diluted in and neutralised by the surrounding residual urine (20 - 30 ml). Thus neither the LMAC nor the pH of the solution constitutes any risk to the patient.

Another advantage lies in the possibility of repeated administration of the antimicrobial compound / -s at chosen doses and intervals without disturbing the integrity of the closed drainage system.

Yet another advantage lies in the kinetics of the LMAC release. There will be a short and high peak of LMAC released in the cuff. The LMACs will quickly permeate the membrane. In contrast, with the use of traditional antibiotics with high molecular weight, as for example in US patent No. 5,417,657, the membrane permeability of the antibiotics will be low, leading to a slow release of the antibiotics. It is well known to a person skilled in the art that a slow release during a long time increases the risk of selection, i.e. that the bacteria develop resistance to antibiotics.

Yet another advantage of the present invention is the *in situ* production of the active substance, the LMAC, when the at least one component and the second component are combined in the cuff. There is no loss of antibacterial activity during transportation or delivery.

A particular advantage lies in the fact that the inventive device can be easily introduced in clinical practice.

The present invention will now be further described in the following non-limiting example.

### Examples

### Example 1. Incubation of an E.coil strain in a solution of ascorbate and sodium nitrate

### 1.1 Materials & methods

An *E. coli* strain isolated from urine of a patient with lower urinary tract infection was used. A 50 ml glass bottle with a narrow neck (Fig. 4) was filled with fresh urine (pH 6.5) from a healthy volunteer. The urine was inoculated with the *E. coli* strain to a final density of 105 colony forming units/ml. Then an all-silicone urinary catheter was Inserted in the bottle and the cuff was filled with a solution comprising:
1. Saline + ascorbate (20 mM) + HCl to a final pH of 2.0 (control; n=3) or
2. Saline + ascorbate (20 mM) + HCl + sodium nitrite (2 mM), pH 2 (nitrite; n=3)

The expanded cuff was fixed at the neck of the bottle to prevent leakage of urine when the bottle was turned up side down. The glass bottle was then incubated in 37° C for 10 h after which growth of E. coli in the surrounding urine was monitored by optical density (OD) at 540 nm on a Spectramax® (Molecular Devices Inc.).

### 1.2. Results

In controls OD values increased from 0.12 to 0.35 while in the nitrite group, no visible growth was observed (OD 0.12 before and 0.13 after10 h).

The experiment shows that the addition of nitrite to an acidic ascorbate solution results in generation of bacteriostatic compounds (such as NO, N₂O₃ and nitrous acid), which can penetrate a thin silicone membrane and inhibit bacteria growing in urine outside the membrane.

### Example 2. Viable counts

### 2. 1 Materials & methods

An *E. coli* isolated from a patient with urinary tract infection and a reference strain, *E. coli* ATCC 25992 were used in this study. An overnight culture was added to 25 ml of urine to a final density of 10⁵ CFU/ml. The urine was placed in long-necked 50 ml flasks with a shape resembling the urinary bladder and the urethra. An all-silicone catheter (Argyle, Sherwood Medical, Tullamore, Ireland) was placed in the flask and the retention balloon was filled with 10 ml of saline containing ascorbic acid (10 mM) and sodium nitrite (5 mM). The pH of the solution was adjusted to 2.5 using hydrochloric acid (3 M). Ascorbic acid and nitrite were prepared and mixed immediately before administration. Ascorbic acid solution alone (pH 2.5) was used as control. After filling the retention balloons the catheter was gently pulled outwards and fixed at the neck whereby the flask opening was sealed off. Then the flasks were turned up side down and incubated at 37 °C for 24 h. At the end of the experiment the urine was serially diluted and further cultured on blood agar plates for determination of viable counts (cfu/ml). All experiments were made in quadruplicate. The pH of urine was 6.5 and did not decrease during the course of the experiments.

In a separate experiment the balloon was emptied and refilled with fresh solution of nitrite and ascorbic acid once daily for 7 days. The incubation was carried out as described above.

### 2.2 Results

In the control experiments using ascorbic acid alone, bacterial counts had increased from 10⁵ to 10⁸ cfu/ml after 24 h. In contrast, when filling the retention balloon with ascorbic acid and nitrite the bacteria were effectively killed.

In the 7 days incubation experiment, no bacteria were detected in the urine.

### Example 3. Drug release kinetics

### 3.1 Materials & methods

The kinetics of NO release from the retention balloon was monitored in separate experiments. The catheter was placed in the flask and after filling the retention balloon with the ascorbic acid/nitrite solution the flask was closed. Synthetic NO-free air was flushed via an inlet at a rate of 4.5 L/min and headspace NO was continuously measured from an outlet by a rapid-response chemiluminescence system (Aerocrine AB, Stockholm, Sweden).

### 3.2 Results

The NO release rate from the balloons peaked initially and then decreased with a half-time of about 30 min, see figure 8. The results prove the concept underlying the invention and show the utility of the device and method for the treatment of urinary tract infections.

### Example 4. Comparative experiment

### 4.1 Materials & methods

The experiment was carried out as described in example 2 with the exception that nitrite and ascorbic acid were replaced by a traditional antibiotic, ciprofloxacin, (Ciloxan®, Alcon Sverige AB, Stockholm, Sweden) Trimetoprim (bacteriostatiskt - hämmar)(Sigma, Stockholm). In a separate flask ciprofloxacin was added directly to the urine.

### 4.2 Results

When ciprofloxacin was placed in the retention balloon no antibacterial effect could be seen in the urine surrounding the balloon. In contrast, when the antibiotic was added directly to the urine, it effectively killed the bacteria as expected. This demonstrates that only a low molecular drug can penetrate the membrane of the balloon.

## Claims

1. An antimicrobial device (1, 7, 12, 22, 22') for insertion in a human or animal body or body cavity, said device having inflatable/expandable means, **characterized in that** said inflatable/expandable means consist of an elastic membrane (5, 10, 26, 26') being permeable to low molecular antimicrobial compounds (LMACs) but impermeable to water, and comprise at least one first component capable of releasing at least one LMAC capable of permeating into the adjacent tissue or body cavity and said at least one first component releases said LMAC upon acidification and the acidification is achieved through the addition of an acidifying component.

2. The device according to claim 1, wherein said at least one first component is nitrite.

3. The device according to claim 1, wherein said acidifying component is acetic acid.

4. The device according to claim 2, wherein said acidifying component is ascorbic acid.

5. The device according to claim 1, wherein said at least one first component is nitrite and said acidifying component is ascorbic acid.

6. The device according to claim 1, wherein the acidifying agent reduces the pH in the device to pH 2-4.

7. The device according to claim 1, wherein said first component and said acidifying component are present in the inflatable/expandable means as dry powders, granulates or thin films.

8. The device according to claim 1, wherein said device is a catheter for insertion into the urinary tract of a human or animal and said inflatable and expandable means comprise an inflatable cuff.

9. The device according to claim 1, wherein said device is an intratracheal tube.

10. The device according to claim 1, wherein said device is a gastric tube.

11. The device according to any one of claim 4 to 6, wherein said at least one LMAC is a reactive nitrogen intermediate.

12. The device according to claim 11, wherein said at least one LMAC is nitric oxide (NO).

13. The device according to any one of the claims above, having a concentration of one or more metal ions in the contents of the inflatable/expandable means or in the material or the surface of said device, said concentration being sufficient to increase the antimicrobial effect.

## Patentansprüche

1. Antimikrobielle Vorrichtung (1, 7, 12, 22, 22') zum Einführen in einen menschlichen oder tierischen Körper oder einen Körperhohlraum, wobei die Vorrichtung ein inflatierbares/expandierbares Mittel aufweist, **dadurch gekennzeichnet, dass** das inflatierbare/expandierbare Mittel aus einer elastischen Membran (5, 10, 26, 26') besteht, die von niedermolekularen, antimikrobiellen Verbindungen (Low Molecular Antimicrobial Compounds - LMACs), jedoch nicht von Wasser durchdrungen werden kann, und die mindestens eine erste Komponente umfasst, die mindestens eine LMAC freisetzen kann, die in das benachbarte Gewebe oder den benachbarten Körperhohlraum eindringen kann und wobei die mindestens eine erste Komponente die LMAC bei Säurebildung freisetzt und die Säurebildung durch die Zugabe einer säurebildenden Komponente erreicht wird.

2. Vorrichtung nach Anspruch 1, wobei die mindestens eine erste Komponente Nitrit ist.

3. Vorrichtung nach Anspruch 1, wobei die säurebildende Komponente Essigsäure ist.

4. Vorrichtung nach Anspruch 2, wobei die säurebildende Komponente Ascorbinsäure ist.

5. Vorrichtung nach Anspruch 1, wobei die mindestens eine erste Komponente Nitrit und die säurebildende Komponente Ascorbinsäure ist.

6. Vorrichtung nach Anspruch 1, wobei das Säuerungsmittel den pH-Wert in der Vorrichtung auf 2 bis 4 pH senkt.

7. Vorrichtung nach Anspruch 1, wobei die erste Komponente und die säurebildende Komponente in dem inflatierbaren/expandierbaren Mittel als Trockenpulver, Granulat oder dünner Film vorhanden sind.

8. Vorrichtung nach Anspruch 1, wobei die Vorrichtung ein Katheter zum Einführen in den Harnweg eines Menschen oder eines Tieres ist und das inflatierbare und expandierbare Mittel eine inflatierbare Manschette umfasst.

9. Vorrichtung nach Anspruch 1, wobei die Vorrichtung ein Intratracheal-Schlauch ist.

10. Vorrichtung nach Anspruch 1, wobei die Vorrichtung ein Magenschlauch ist.

11. Vorrichtung nach einem der Ansprüche 4 bis 6, wobei die mindestens eine LMAC ein reaktives Stickstoff-Zwischenprodukt ist.

12. Vorrichtung nach Anspruch 11, wobei die mindestens eine LMAC Stickoxid (NO) ist.

13. Vorrichtung nach einem der vorhergehenden Ansprüche, die eine Konzentration von einem oder mehreren Metallionen in den Inhalten des inflatierbaren/expandierbaren Mittels oder in dem Material oder auf der Oberfläche der Vorrichtung aufweist, wobei die Konzentration ausreicht, um die antimikrobielle Wirkung zu verstärken.

## Revendications

1. Dispositif antimicrobien (1, 7, 12, 22, 22') destiné à être introduit dans le corps ou dans une cavité corporelle d'un sujet humain ou animal, ledit dispositif possédant des moyens gonflables/expansibles, **caractérisé en ce que** lesdits moyens gonflables/expansibles se composent d'une membrane élastique (5, 10, 26, 26') qui est perméable aux composés antimicrobiens de poids moléculaire faible (LMAC, *low molecular antimicrobial compounds*) mais imperméable à l'eau, et **en ce qu'**ils comprennent au moins un premier composant capable de libérer au moins un LMAC capable de diffuser dans le tissu adjacent ou la cavité corporelle et ledit premier composant libère ledit LMAC après une acidification, laquelle acidification est obtenue par l'ajout d'un composant acidifiant.

2. Dispositif selon la revendication 1, dans lequel ledit au moins un premier composant est un nitrite.

3. Dispositif selon la revendication 1, dans lequel ledit composant acidifiant est l'acide acétique.

4. Dispositif selon la revendication 2, dans lequel ledit composant acidifiant est l'acide ascorbique.

5. Dispositif selon la revendication 1, dans lequel ledit au moins un premier composant est un nitrite et ledit composant acidifiant est l'acide ascorbique.

6. Dispositif selon la revendication 1, dans lequel l'agent acidifiant abaisse le pH dans le dispositif à pH 2-4.

7. Dispositif selon la revendication 1, dans lequel ledit premier composant et ledit composant acidifiant sont présents dans les moyens gonflables/expansibles sous la forme de poudres sèches, de granulés ou de films minces.

8. Dispositif selon la revendication 1, dans lequel ledit dispositif est un cathéter destiné à être introduit dans le système urinaire d'un sujet humain ou animal et lesdits moyens gonflables et expansibles comprennent un bourrelet gonflable.

9. Dispositif selon la revendication 1, dans lequel ledit dispositif est une sonde endotrachéale.

10. Dispositif selon la revendication 1, dans lequel ledit dispositif est une sonde gastrique.

11. Dispositif selon l'une quelconque des revendications 4 à 6, dans lequel ledit au moins un LMAC est un intermédiaire azoté réactif.

12. Dispositif selon la revendication 11, dans lequel ledit au moins un LMAC est l'oxyde nitrique (NO).

13. Dispositif selon l'une quelconque des revendications précédentes, ayant une concentration en un ou plusieurs ions métalliques dans le contenu des moyens gonflables/expansibles ou dans le matériau ou la surface dudit dispositif, ladite concentration étant suffisante pour augmenter l'effet antimicrobien.
